# EUROPEAN PATENT APPLICATION

(11) **EP 1 637 524 A1**
(43) Date of publication of application: **22.03.2006**
(21) Application number: 04022336.4
(22) Date of filing: 20.09.2004
(51) Int. Cl.: C07D 257/02, G01N 24/08, C07D 401/12

(54) **10-substituted 1-, 4, 7,-tris (carboxymethyl)-1,4,7,10-tetraazacyclo-dodecane derivative for use as contrast agents**

(71) Applicant: Max-Planck-Gesellschaft zur Förderung der Wissenschaften e.V., 80539 München (DE)
(72) Inventor: Mishra, Anil K., 72076 Tuebingen (DE)
(74) Representative: Weiss, Wolfgang

(57) **Abstract**

The present invention relates to smart chelating agents suitable for forming stable complexes with lanthanides and/or transition metals.

## Description

The present invention relates to smart chelating agents suitable for forming stable complexes with lanthanides and/or transition metals.

Chelating agents are polydentate ligands which bind with metal ions to form coordination compounds. Such metal ligand complexes are of great practical importance and find numerous applications in the field of medicine. For example, chelating agents are suitable as chemical decontamination and decorporation agents, MRI contrast agents, for radiopharmaceutical applications, luminescent probes and *in vivo* NMR shift reagents.

Often, metals or metal ions used in medical applications are toxic and thus the major concern with the use of metal chelates *in vivo* is the potential release of the toxic heavy metal ion from the complex. It is therefore important, that the chelating agents form kinetically inert and thermodynamically stable complexes in order to ensure the innocuousness of the agent during its travel through the body of a patient.

The biological distribution of agents is important. Preferably contrast agents localize in one tissue type in preference to other tissues in order to highlight the pathology of the target tissue.
Further, it is desirable, to have chelating agents at one's disposal, the properties of which can be altered. Thus, for example, the effect of temperature, pH, O₂ pressure, enzymes and metal ion concentration on the binding of a chelating agent can be utilised to affect the properties of coordination compounds. However, coordination compounds currently used are often non-specific.

Therefore functionalized ligands have been developed having responsive groups which act as reporter moieties or which can be used to attach covalently to biological molecules constituting targeted agents. Via a responsive group it is possible to regulate the properties of the resulting coordination compounds as a function of particular chemical variables.

For example, Silvio et al., *Chem. Comm.,* 1999, 1577-1578 discloses the use of pH-responsive gadolinium complexes for MRI applications. Meade et al., *J. Am. Chem. Soc.,* 1999, 121, 1413-1414 describes the use of DOPTA-Gd contrast agents for use in MRI, whose relaxivity is selectively modulated by the Ca²⁺ concentration. In these chelating agents an amide group is used as the responsive group.

As is described in the literature (Aime et al., *Chem. Eur. J*., 2000, 6, 2609-2617), the amide oxygen is needed to ensure the stability of a gadolinium complex. The disadvantage associated with the amide group is that it will be chopped by enzymes like carboxypeptidases and it will no longer be a responsive agent for binding with positively charged polyamino acids and/or it will not recognise any precursors of phosphonate to alter the relaxivity.

There is thus an ongoing need for new compounds with improved performances. In particular, there is a need for chelating agents capable of forming stable complexes with lanthanides and transition metals.

The macrocyclic ligand 1,4,7,10-tetraazacyclododecane (DOTA) is a preferred structure to base a new class of chelating agents. The synthetic versatility of the macrocyclic skeleton provides an ideal candidate for the synthesis of smart chelating agents.

The present invention provides novel chelating agents based on 1,4,7,10-tetraazacyclododecane, bearing a responsive group on the tenth position of the ring.

The present invention provides novel chelating agents of Formula I 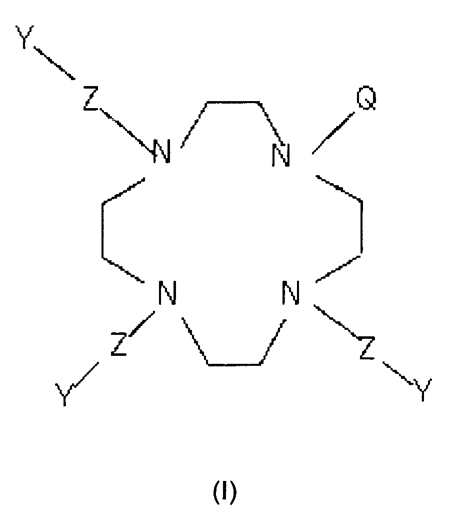 wherein
wherein:
Q is a responsive group capable of reversible chelating metal atoms and/or ions, which is not hydrolyzable under physiological conditions,
Z is independently a substituted or unsubstituted straight or branched chain alkylene or alkenylene group, and
Y is in each case independently an acidic group, preferably selected from the group consisting of CO₂H, PO₃H₂, PO₄H₂, PO₃H, SO₄H and SO₃H,
or salts thereof, particularly salts with pharmaceutically acceptable counter ions.

The functional group Y is preferably CO₂H or CO₂⁻, however it may as well be any other acidic residue. Y is covalently attached to the DOTA macrocycle via Z. Z may for example be a C₁₋₄ alkylene or C₂₋₄ alkenylene group, which may be substituted as defined below, e.g. with halo, C₁₋₄ alkyl and/or C₂₋₅ alkenyl groups. Preferably Z is CH₂.

According to the invention, the DOTA macrocycle may optionally be substituted on one or more of the carbon ring positions as defined below, e.g. with halo, C₁₋₄ alkyl and/or C₂₋₄ alkenyl groups.

In the context of the present invention, Q is referred to as a responsive group. The responsive group is capable of binding to metal ions. The binding properties of Q, however, depend on the environmental conditions as explained below. Preferably Q comprises a linking group and a functional group X which is defined as Y above.

According to the invention, Q is not hydrolyzable unter physiological conditions and preferably does not contain a carboxamido group. Thus, the properties of the responsive group are preserved in the presence and absence of target molecules/ions, e.g. enzymes, and the ability to act as a responsive agent is maintained.

According to the present invention the binding properties of Q depend on environmental conditions, particularly temperature, pH, the presence of enzymes, the presence of cations (e.g. Ca²⁺), and/or polyamino acids, and/or oxygen pressure. Thus, variation of such parameters can influence the ability of the chelating agent to form coordination compounds.

The functional group X, preferably PO₃H₂, of Q is covalently attached to the DOTA macrocycle via a linking group. According to the invention, "linking group" refers to any bivalent moiety which connects X and the nitrogen atom of the macrocycle.

Preferably, the linking group contains one or more heteroatoms selected from the group of N, O, P and S, wherein the heteroatom may serve as electron donating moiety such that the linking group contributes to the binding to metal atoms and/or ions. Particularly suitable are electron rich heteroatoms having free electron pairs and/or ionizable groups.

According to a preferred embodyment of the invention, Q contains a cyclic group. The cyclic group may be any substituted or unsubstituted ring system e.g. a mono or oligocycle. Suitable cyclic groups include up to 20 carbon atoms and 0-4 hetero atoms selected from the group of N, S, and O. Preferred cyclic groups are aryl or heteroaryl groups.

According to a further preferred embodyment of the invention, Q contains at least one double bond. More preferably Q contains a C=N bond.

In general, substituents for the DOTA macrocycle and groups Q and Z are preferably selected from OH, halo, e.g. F, Cl, Br or I, amino, carboxyl, sulfo, substituted or unsubstituted alkyl with up to 10 carbon atoms (e.g. methyl), C₁-₆alkoxy, C₆₋₁₄ aryloxy, C₆₋₁₄ arylthio, C₃₋₆ alkenyl, C₃₋₆ alkinyl, C₃₋₆ acyl, C₃₋₆ alkoxycarbonyl, C₆₋₁₄ carbamoyl, C₇₋₁₄ aralkyl, C₆₋₁₄aryl and/or C₆₋₁₄ heteroaryl. Further, substituents capable of coupling to biomolecules may be present.

The responsive group of the chelating agent of the invention is preferably represented by formula (II)

-(CR¹₂)ₙ-CR²=N-A-(CR³₂)ₚ-O-X (II)

wherein n and p are independently integers from 0 to 5, wherein n is preferably 2 and/or p is preferably 1,
R¹, R² and R³ independently represent H, OH, amino, carboxy, sulfo, substituted or unsubstituted alkyl with up to 10 carbon atoms (e.g. methyl), C₁₋₆ alkoxy, C₆₋₁₄ aryloxy, C₆₋₁₄ arylthio, C₃₋₆ alkenyl, C₃₋₆ alkinyl, C₃₋₆ acyl, C₃₋₆ alkoxycarbonyl, C₆₋₁₄ carbamoyl, C₇₋₁₄ aralkyl, C₆₋₁₄ aryl or C₆₋₁₄ heteroaryl, wherein R¹, R² and/or R³ preferably are H,
X is as defined for compound (I) and preferably -PO₃H₂, and
A represents a substituted or unsubstituted aliphatic unsaturated, or aromatic mono or oligocyclic ring system with up to 20 carbon atoms and 0-4 hetero atoms selected from the group of N, S, and O. Preferred rings are for example benzene, naphthene, and thiophen. A particularly preferable heterocycle is pyridin, which may optionally be substituted.

A particularly preferable chelating agent of the invention is the compound represented by formula III or a salt thereof:

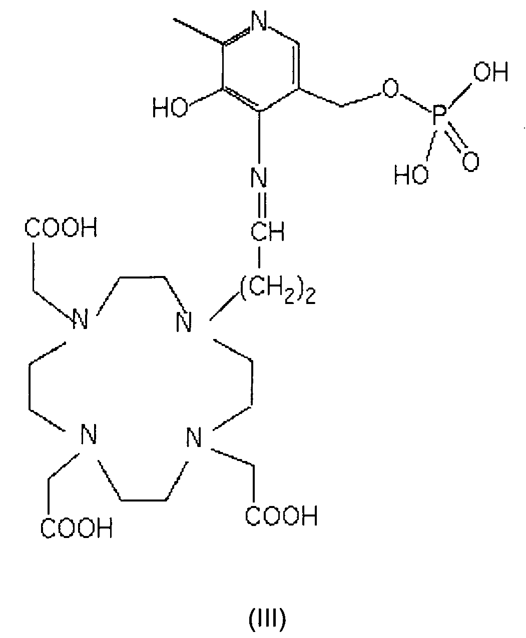

According to a further aspect the present invention relates to a metal chelate wherein a chelating agent of formula I is coordinated to a metal atom or ion. The metal atoms or ions are preferably polyvalent metal atoms or ions, e.g. heavy metals capable of chelating, e.g. transition metals, lanthanides or actinides, such as iron, copper, manganese, chromium, erbium, europium, dysprosium, holmium and gadolinium. If the chelate is used for diagnostic applications, the metal preferably is a detectable metal, e.g. a paramagnetic or superparamagnetic metal, a radioactive, or a fluorescent metal. Most preferably the metal is gadolinium.

In the metal chelates of the present invention, the responsive group Q may be attached to the metal centre. Depending on the environmental conditions Q dissociates from the metal centre. For example, Q can dissociate from the metal centre in the presence of competing positively charged ions such as Ca²⁺ , polyamino acids, etc. and/or enzymes. Additionally, the binding properties of Q can be altered by a change in the pH.

According to one aspect of the invention the binding of the responsive group Q depends on the presence of physiological metal ions such as Ca²⁺, Mg²⁺, Cu²⁺, Fe²⁺ and/or Zn²⁺. Such metal cations compete with the metal centre for the group Q. In the presence of such competing cations Q gets dissociated from the metal centre and coordinates to these physiological cations instead. It is preferred, that the responsive group present in the componds of the invention selectively binds to one particular type of physiological cation, e.g. to Ca²⁺.

In a further aspect of the invention, the chelating agent is coupled to a biomolecule, e.g. a protein, a peptide, a nucleic acid such as DNA or RNA, a saccharide, a lipid, or a non-peptidic modulator such as a hormone etc. The coupling may be effected by known methods, e.g. by coupling a reactive group on the compound (I) with a reactive group on the biomolecule optionally via a bifunctional linker. Respective methods for coupling of polyazamacrocycles to biomolecules are known in the art and e.g. disclosed in: (a) Sundberg, M.M., Meares, C.F., Goodwin, D.A., and Diamanti, C.T., Chelating agents for the binding of metal ions to macromolecules, Nature, 1974, 250, 587-588 and (b) Despandee, S.V., DeNardo, S.J., Kukis, D.L., Moi, M.K., McCall, M.J., DeNardo, G.L., and Meares, C.F., Yttrium-90 labeled monoclonal antibodies for therapy: Labeling by a new macrocyclic bifunctional chelating agent, J. Nucl. Med., 1990, 31, 473-479.

The coupling to the biomolecule may occur via the responsive group Q or via another group of the compound. If the coupling occurs via Q, the coupling may modify the responsive properties. Thus, in the absence of certain biological molecules Q is coordinated to the metal centre whereas in the presence of for example positively charged polyaminoacids Q gets dissociated from the metal.

According to a further aspect, the present invention relates to conjugates comprising a metal chelate of the invention and a biomolecule.

Even though under certain environmental conditions Q no longer participates in the binding to the chelated metal centre, the remaining ligand functionalities of the polydentate ligands of the invention (4 N of the tetraazacyclododecane macrocycle and 3 Y (e.g. carboxylate groups)) occupy seven coordination sites of the metal centre, which results in very stable complexes. Thus, there is no danger of release of the possibly toxic metal even under physiological conditions.

Due to the above mentioned possibilities of altering the binding properties of Q towards chelated metals, the chelating agents, metal chelates, and/or conjugates of the invention can be used for preparing metallopharmaceuticals. It is possible to use said compounds as contrast agents in medical imaging technologies such as magnetic resonance imaging (MRI), gamma or positron emission tomography, optical or fluorescence microscopy, for example.

Used as contrast agents, the metal chelates of the invention are able to generate a signal dependent on some variable in their immediate environment. With regard to MRI, the number of water molecules in the first coordination sphere, the water exchange rate and the rotational correlation time have a strong effect on the relaxivity of the compounds and can be influenced by many factors. The responsive group of the compounds of the invention is capable to modulate access to a chelated metal ion dependent on several stimuli present in their environment.

Metal chelates of paramagnetic and superparamagnetic metals are of particular interest for application as contrast agents in MRI. Gadolinium chelates are especially useful, because Gd³⁺ has a very high magnetic moment (µ² = 63 µ_{B}²), and a symmetric ground state, ⁸S_{7/2}.

According to one aspect of the invention, the relaxivity of the contrast agents of the invention can be selectively modulated by the concentration of cations such as copper, calcium and iron. In the absence of such additional cations, Q may interact with the metal centre e.g. through ionic interactions. In the presence of cations, Q will rearrange to bind said cation. Thereby, the coordination sphere of the metal is altered and access of e.g. water to the chelated metal is facilitated. It is preferred, that the contrast agent is selective for binding to one particular cation (e.g. Ca²⁺).

According to another aspect of the invention, the properies of the contrast agents of the invention are dependent upon the pH. In solution, the dependencies of the properties (e.g. relaxivity) of metal chelates on pH may be due to variation in the hydration spere of the metal chelate. Preferably, the compounds of the invention are strongly pH dependent through structural changes which limit the internal mobility of the chelate moieties.

Furthermore, targeted delivery to specific cells or tissue of the metal chelates according to the present invention is possible since the responsive group Q may be capable of selectively binding to biological molecules. Thus, the metal chelates of the invention are useful as targeted contrast agents.

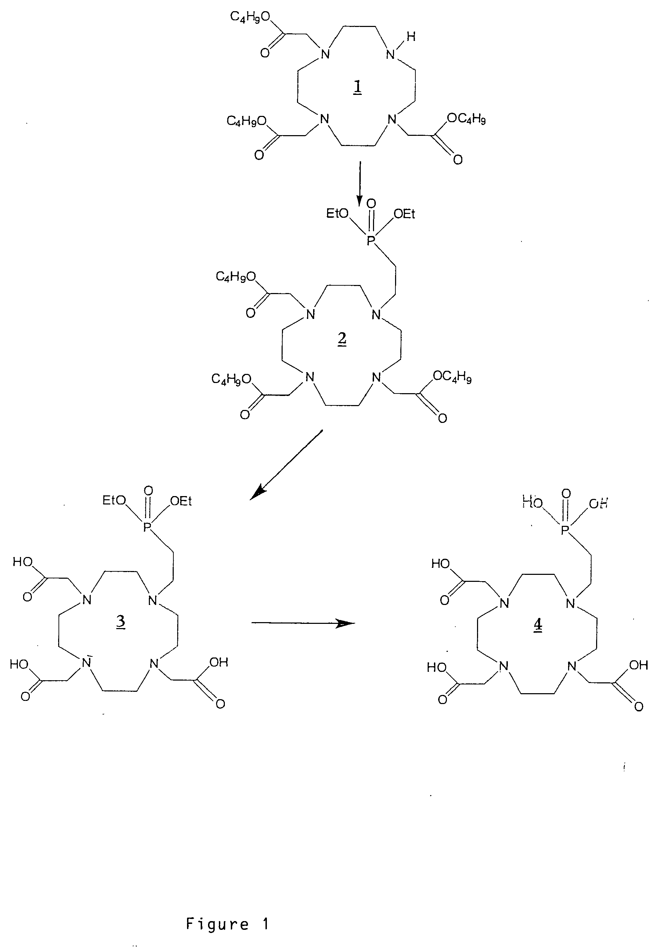

**Figure 1:** reaction scheme of the synthesis of 1,4,7-tris(carboxymethyl)-10-[N-2(diethyl-phosphono-ethyl)]-tetraazacyclododecane **(4)**

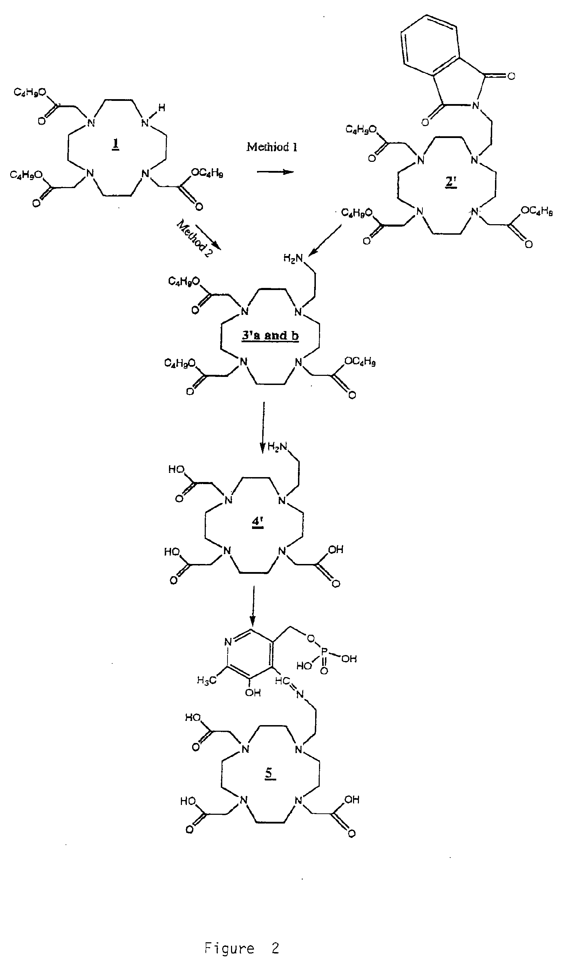

**Figure 2:** reaction scheme of the synthesis of 1,4,7-tris(carboxymethyl)-10-{2-((3-hydroxy-2-methyl-5-phosphono-oxy-methyl-pyridin-4-ylmethylene)-amino]-ethyl}-1,4,7,10-tetraazacyclododecane **(5)**
**Figure 3:** graphs showing the results of relaxivity measurements:
   a) T₁ values measured at various pH for variing concentrations of Ca²⁺
   b) r depending on the pH

The following examples are provided to more fully illustrate the present invention but are not intended to be, nor should they be construed as being limiting in any way of the scope of the invention.

### Examples

### 1. Synthesis of 1,4,7-tris(carboxymethyl)10-[N-2(diethyl-phosphono-ethyl)]-tetraazacyclododecane (4).

### 1.1. Preparation of 1,4,7-tris(carbobutoxymethyl)-1,4,7,10-tetraaza-cyclododecane (1)

A solution of 2.20 equivalents of *tert*-butylbromoacetate in CHCl₃ (40 ml) was added dropwise to a solution of one equivalent of tetraazacyclododecane in in CHCl₃ and the solution was stirred at room temperature for 72 hours. The free protonated tetraazacyclododecane was then removed by filtration, and the resulting filtrate was washed with water and dried over anhydrous Na₂SO₄, filtered, and evaporated under reduced pressure yielding 72% of tri-N-substituted tetraazacyclododecane **1,** which was used for the next step without further purification.

**ESI-MS(+):** found: 515 [M+H]⁺; calculated for C₂₆H₅₀N₄O₆: m/z 514, Rf = 0.64 (CHCl₃/MeOH: 5:1), ^{**1**}**H NMR** (250 MHz, CDCl₃): 1.45 (s, 27H), 2.17 to 2.81 (m, 16H), 3.33 (s, 6H), 10.01 (br, 1 H); ^{**13**}**C NMR** (CDCl₃): 28.61 (C-16); 48.08 (C-9, C-11); 50.69 (C-2, C-6); 52.32(C-8, C-12); 53.02 (C-3, C-5); 57.21 (C-13); 80.74 (C15); 171.25, 171.41 (C-14a, C-14c). Elemental analysis: calculated (found) for: C₂₆H₅₀N₄O₆; C, 60.67(60.69); H, 9.79 (9.81); N, 10.89 (10.87); O, 18.65 (18.71).
**MS-MS of 515 in ESI mode: (El/intensity in %) (Scheme 2):** m/z= 459 (100%); 409 (88%), 347 (20%).

### 1.2. reparation of 1,4,7-tris(carbobutoxymethyl)-10-[N-2(diethyl-phoshono-ethyl)]-1,4,7,10-tetraazacyclododecane (2)

1.1 equivalents of diethyl 2-bromoethyl phosphonate in N, N-dimethyformamide (DMF) was added to a solution of 1 and 3 equivalents of powdered anhydrous K₂CO₃ in DMF and the reaction mixture was heated to 100-110°C within 30 minutes. The reaction mixture was allowed to stir at 100-110°C for 26 h under a N₂ atmosphere and the reaction was monitored by ESI-MS. After completion of the reaction, the reaction mixture was cooled to room temperature, filtered, and evaporated under reduced pressure. The product was dissolved in ethylacetate and washed several times with 100mL of water and the organic phase was dried over anhydrous Na₂SO₄, filtered and evaporated to dryness, yielding 93% of 2.

**ESI-MS(+):** found: 701 [M+Na]⁺; calculated for C₃₂H₆₃N₄O₉P: m/z 678. ^{**1**}**H NMR (250** MHz CDCl₃): 1.35 (t, 3H), 1.45(s, 27H), 1.87 (mixed d and t, 2H), 2.41 (t, 2H), 2.51 to 3.29 (m, 22H), 4.07 (q, 2H); ^{**13**}**C NMR** (CDCl₃): 14.79 (C-20); 23.01 (C-18); 28.81 (C-16); 48.08 (C-17); 50.10 (C9, C-11); 52.4 to 52.7 (C-2 to C-6); 56.62 (C-8, C-12); 57.21 (C-13); 62.3 (C-19); 81.79 (C-15); 169.59 (C-14); ^{**31**}**P NMR** (CDCl3): 18.52. Elemental analysis: calculated (found) for C₃₂H₆₃N₄O₉P: C, 56.62 (56.67); H, 9.35 (9.39); N, 8.25 (8.30); O, 21.21 (21.26); P, 4.56 (4.12).

### 1.3. Preparation of 1,4,7-tris(carboxymethyl)-10-[N-2(diethyl-phoshono-ethyl)1-1,4,7,10-tetraazacyclododecane (3)

The carbobutoxy derivative **2** (0.295 mmole) was dissolved in neat trifluoroacetic acid (2mL) with stirring at 0°C (pH 2) for 2 h and the reaction mixture was stirred for an additional 6 hours at ambient temperature; the reaction was monitored by ESI-MS. After completion of the reaction the pH was adjusted to 6 using 6M HCl and water was removed to dryness. The obtained glassy solid was redissolved in one liter of water and the product was lyophilized yielding 80% of **3.**

**ESI-MS(+):** found: 511 **[M+H]**^{**+**}**;** calculated for C₂₀H₃₉N₄O₉P: m/z 510. ^{**1**}**H NMR** (D₂O): 1.35 (t, 3H), 1.87 (mixed d and t, 2H), 2.41 (t, 2H), 2.51 to 3.29 (m, 22H), 4.07 (q, 2H);^{**13**}**C NMR** (D₂O): 14.79 (C-18); 23.12 (C-16); 48.01 (C-15); 50.10 (C9, C-11); 52.4 to 52.7 (C-2 to C-6); 56.62 (C-8,C-12); 57.21 (C-13); 62.32 (C-17); 173.59 (C-14); ^{**31**}**P NMR** (CDCl₃): 18.52. Elemental analysis: calculated (found) for C₂₀H₃₉N₄O₉PTFA: C, 42.31 (42.35); H, 6.46 (6.48); F, 9.13 (9.15); N, 8.97 (9.01); O, 28.18 (28.22); P, 4.96 (4.65).

### 1.4. Preparation of 1,4,7-tris(carboxymethyl)-10-[N-2(phosphonoethyl)]1-1,4,7,10-tetraazacyclododecane (4)

The diester compound **3** was converted to the sodium salt by treatment with 1 M aqueous ethanolic sodium hydroxide. The sodium salt of **4** was loaded on cation exchange resin DOWEX 50W yielding quantitative replacement of the sodium ions by hydrogen ions.
**ESI-MS(+):** found: 455 [M+H]⁺; calculated for C₁₆H₃₁N₄O₉P: m/z 454. ^{**1**}**H NMR** (D₂O): 1.87 (mixed d and t, 2H), 2.41 (t, 2H), 2.51 to 3.29 (m, 22H); ^{**31**}**P NMR** (CDCl₃): 18.52. Anal. Calculated (found) for C₁₆H₃₁N₄O₉P: C, 42.29 (42.18); H, 6.88 (6.79); N, 12.33 (12.41); O, 31.69 (31.71); P, 4.96 (4.75).

### 2. Synthesis of 1,4,7-tris(carboxymethyl)-10-[N-2(ethyl)phthalimide-1,4,7,10-tetraazacyclododecane 2'(5).

### Method 1:

### 1,4,7-tris(carbobutoxymethyl)-10-[N-2(ethyl)phthalimide]-1,4,7,10-tetraazacyclododecane (2'):

One equivalent of N-(bromoethyl) phthalimide in DMF was added to a solution of **1** and 2.5 equivalents of K₂CO₃ in DMF and the reaction mixture was heated to 100-110°C for 12 h under N₂ atmosphere. The reaction mixture was cooled to room temperature, filtered, and evaporated under reduced pressure. The product was dissolved in chloroform and washed several times with 100mL of water and the organic phase was dried over anhydrous Na₂SO₄, filtered and evaporated to dryness, yielding 90% of 1,4,7-tris(carbobutoxymethyl)-10-[N-2(ethyl)phthalimide]-1,4,7,10-tetraazacyclododecane **2'.**

**ESI-MS(+):** found: 701 [M+Na]⁺; calculated for C₃₆H₅₇N₅O₈: m/z 678. ^{**1**}**H NMR** (250 MHz CDCl₃): 1.45 (s, 27H), 2.51 to 3.29 (m, 24H), 3.58 (t, 2H), 7.72 (dd, 2H, JH-H= 3.1), 7.83 (dd, 2H, JH-H = 6.0); ^{**13**}**C NMR** (CDCl₃): 28.81 (C-18); 29.01, 39.21, 47.32, 49.2, 51.29, 57.01, 60.71, 123.40, 131.72, 134.23, 167.71, 170.34, 171.15. Elemental analysis: calculated for C₃₆H₅₇N₅O₈: C, 59.62; H, 7.51; N, 11.60; O, 21.20; Found: C, 59.66; H 7.55; N, 11.54; O, 21.35.

### 1,4,7-tris(carboxymethyl)-10-(2-aminoethyl)-1,4,7, 10-tetraazacyclododecane (3'a):

**2'** (0.70 mmol) was treated with a mixture of 48% HBr (100 mL) and glacial acetic acid (100 mL) pH ~2.5 to 3.0 and the resulting mixture was refluxed for 12h. After evaporation to dryness the solid residue was treated with 1 M HBr (100 mL) and the undissolved residue of phthalic acid was removed by filtration. The filtrate was evaporated to dryness and redissolved in hot 48% HBr (75 mL). On gradual addition of glacial acetic acid (200 mL) to the stirred and ice-cooled solution the crude amine hydrobromide precipitated which was collected, washed thoroughly with ethanol and ether, and finally dried over P₄O₁₀ at 1 mm-Hg for several hours. The amino derivative was dissolved in trifluoroacetic acid ( 3mL) at pH 2 with stirring at 0°C for 2 h and the reaction mixture was stirred for an additional 6 hours at ambient temperature, the reaction mixture was monitored by LC-MS. After completion of the reaction the pH was adjusted to 6 using 6M HCl and water was removed to dryness. The obtained glassy solid was redissolved in one liter of water and the product was lyophilized. Yield: 80%. Reverse-phase C₁₈ HPLC: solvent A, 0.1 M ammonium acetate, pH 6; solvent B, MeOH; 15-65% B, 0-25 min., 65-100% B, 30-35 min.; 100-15% B, 35-40 min.; product peak 6 min.
**ESI-MS(+):** found: 390 [M+H]⁺; calculated for C₁₆H₃₁N₅O₆: m/z 389. ^{**1**}**H NMR** (D₂O): 1.35 (t, 3H), 1.87 (mixed d and t, 2H), 2.41 (t, 2H), 2.51 to 3.29 (m, 22H), 4.07 (q, 2H); ^{**13**}**C NMR** (D₂O): 23.12 (C-18); 48.01 (C-17); 50.10 (C9, C-11); 52.4 to 52.7 (C-2 to C-6); 56.62 (C-8,C-12); 57.21 (C-13); 173.59 (C-14). Elemental analysis: Calculated (found) for: C₁₆H₃₁N₅O₆:C, 49.34 (49.37); H, 8.02 (8.09); N, 17.98 (18,01); O, 24.64 (24.71).

### Method 2:

### 1,4,7-tris(carbobutoxymethyl)-10-(2-aminoethyl)-1,4,7,10-tetraazacyclododecane (3'b):

One equivalent of 2-bromoethylamine hydrobromide in DMF was added to a solution of **1** and **4** equivalents of K₂CO₃ in DMF and the reaction mixture was heated to 100-110°C for 26 h under N₂ atmosphere. The reaction mixture was cooled to room temperature, filtered, and evaporated under reduced pressure. The product was dissolved in chloroform and washed several times with 100mL of water and the organic phase was dried over anhydrous Na₂SO₄, filtered and evaporated to dryness, yielding 80% of 1,4,7-tris(carbobutoxymethyl)-10-(2-aminoethyl)-1,4,7,10-tetraazacyclododecane **3'b.**
**ESI-MS(+):** found: 581 [M+Na+H]⁺; calculated for C₂₈H₅₅N₅O₆: m/z 557. ^{**1**}**H NMR** (250 MHz CDCl₃):1.45(s, 27H), 2.48(br, NH2), 2.51 to 3.29 (m, 24H), 3.58(t, 2H). Elemental analysis: calculated (found) for C₂₈H₅₅N₅O₆: C, 60.29 (60.31); H, 9.94(10.01); N, 12.56(12.61); 017.21 (17.26).

### 1,4,7 tris(carbobutoxymethyl)-10-{2-[(3-hydroxy-2-methyl-5-phosphonooxy-methyl-pyridin-4-ylmethylene)-amino]-ethyl}-1,4,7,10-tetraazacyclododecane (4'):

To a solution of pyridoxal 5-phosphate (alkaline CH₃OH) a methanolic solution of 1,4,7-tris(carbobutoxymethyl)-10-(2-aminoethyl)-1,4,7,10-tetraazacyclododecane **(3'b)** was added slowly at 4°C. The reaction mixture was stirred at ambient temperature (25°C) for 4 hours and the reaction was monitored by mass spectrometry. After completion of the reaction the desired product was filtered and dried under reduced pressure yielding 85% of 1,4,7 tris(carbobutoxymethyl)-10-{2-[(3-hydroxy-2-methyl-5-phosphono-oxy-methyl-pyridin-4-ylmethylene)-amino]-ethyl}-1,4,7,10-tetra-azacyclo-dodecane **4'.** The remaining portion was recovered by evaporating the filtrate to dryness.

**ESI-MS(-):** found: 783-785 isotopic distribution in negative mode [M-3H]⁺; calculated for C₃₆H₆₃N₆O₁₁P: m/z 786-788. ^{**1**}**H NMR** (250 MHz CDCl₃): 1.45 (s, 27H), 2.51 to 3.29 {mixed multiplet, 27H (-CH₂-N-CH₂-N- 16H ), (-CH₂₋CO₂-8H) and (-CH₃-Py-3H)} 3.58 (t, 2H), 3.94 (s, 1 H, -N=CH-Py), 4.53 (d, 2H, CH₂OP, J_{PH} = 3.93Hz), 7.42 (s, 1 H, Py-H). Elemental analysis: calculated (found) for C₃₆H₆₃N₆O₁₁P: C, 54.95(55.02); H, 8.07(8.11); N, 10.68(10.72); O, 22.37(22.43); P, 3.94(3.72).

### 1,4,7-tris(carboxymethyl)-10-{2-[(3-hydroxy-2-methyl-5-phosphono-oxymethyl-pyridin-4-ylmethylene)-amino]-ethyl}-1 1,4,7, 10-tetraazacyclododecane (5):

The carbobutoxy derivative **4'** was dissolved in trifluoroacetic acid (3mL) at (pH 2) with stirring at 0°C for 2 h and the reaction mixture was stirred for an additional 6 hours at ambient temperature, the reaction mixture was monitored by LC-MS. After completion of the reaction the pH was adjusted to 6 using 6M HCl and water was removed to dryness. The obtained glassy solid was redissolved in one liter of water and the product was lyophilized. Yield 80%. Reverse-phase C₁₈ HPLC: solvent A, 0.1 M ammonium acetate, pH 6; solvent B, MeOH; 15-65% B, 0-25 min., 65-100% B, 30-35 min.; 100-15% B, 35-40 min.; product peak 18min.

**ESI-MS:** found: 641 [M+Na]⁺; calculated for C₂₄H₃₉N₆O₁₁P: m/z 618: ^{**1**}**H NMR** (250 MHz D₂O) 2.51 to 3.29{mixed multiplet, 27H (-CH₂-N-CH₂-N- 16H ), (-CH₂-CO₂-8H) and (-CH₃-Py-3H)} 3.58 (t, 2H), 3.94 (s, 1 H, -N=CH-Py), 4.53 (d, 2H, CH₂OP J_{PH} = 3.93Hz), 7.42 (s, 1 H, Py-H). Elemental analysis: calculated (found) for C₂₄H₃₉N₆O₁₁P: C, 46.60 (46.65); H, 6.35 (6.41); N, 13.59 (13.62); O, 28.45(28.51); P, 5.01 (4.74).

### 3. Preparation of Gadolinium complexes of 4 and 5

The gadolinium complexes of **4** and **5** were prepared by mixing a slight excess (5 to 10%) of chelating agents **4** and **5** with hexahydrated gadolinium hydrochloride. The pH was adjusted to 6-7. The absence of free gadolinium ions was checked with xylenol orange indicator. Unbound chelating agents were removed using Sep-Pak cartridge C-18 reverse phase (Agilent, Karlsruhe, Germany).

### 4. Relaxivity measurements

Four different concentrations 0.1, 0.4, 0.7 and 1.0 mM of 0.4 mL contrast agent were prepared in PE tubes in 50mM NaCl solution. The pH dependence of the relaxivity was measured for a series of pH from 4-9 in 0.5 pH steps. The pH was adjusted to the values from 4-9 using 17M glacial acetic acid and Na₂CO₃, respectively.

The dependence of r1 on the concentration of Ca²⁺ was measured for a concentration range from 10⁻⁹ to 10⁻³ M at different pH including physiological pH. The measurements of the proton r₁ and r₂ relaxivities of the Gd-compounds were performed at 300 MHz on a vertical 7 Tesla/ 60cm Biospec MR imaging system (Bruker Biospin, Ettlingen, Germany). The 38-cm inner diameter gradient insert reaches 82 mT/m in 130 µs using a 750V/500A gradient amplifier. An 18-cm volume coil was used for radiofrequency transmission and reception. Up to 52 tubes could be measured simultaneously corresponding to 13 conditions with four different concentrations of the contrast agent. The temperature of the samples was maintained at 21 °C or 38 °C by heated air flow with an accuracy of +/-0.1°C.

T₁ measurements were performed with a saturation recovery spin echo sequence (field-of-view 12 cm, matrix 256x256, slice thickness 5 mm, TE 14 ms, TR 50-8000 ms in 32 logarithmic-spaced steps). T₂ measurements were done with a multi-echo CPMG sequence (TE 14-896 ms, 64 echoes, TR 10 s). The image series were voxelwise fitted to a monoexponential decay (three parameters) using NLLS with the Gauss-Newton method (MATLAB 6.5 R13, Mathworks, Inc.). To extract the decay constants R_{1,2}=1/T_{1,2} from the voxels of one tube, the distribution of the ensemble was fitted to a Gaussian and reported as standard deviation. The ensemble error matched closely the errors of a single-voxel fit, which shows that no further systematic errors were introduced by the image encoding. Finally, the relaxivity r_{1,2} was calculated from the slope of R_{1,2}(*C*) versus the concentration c of the contrast agent by an error-weighted linear regression.

### 5. Results

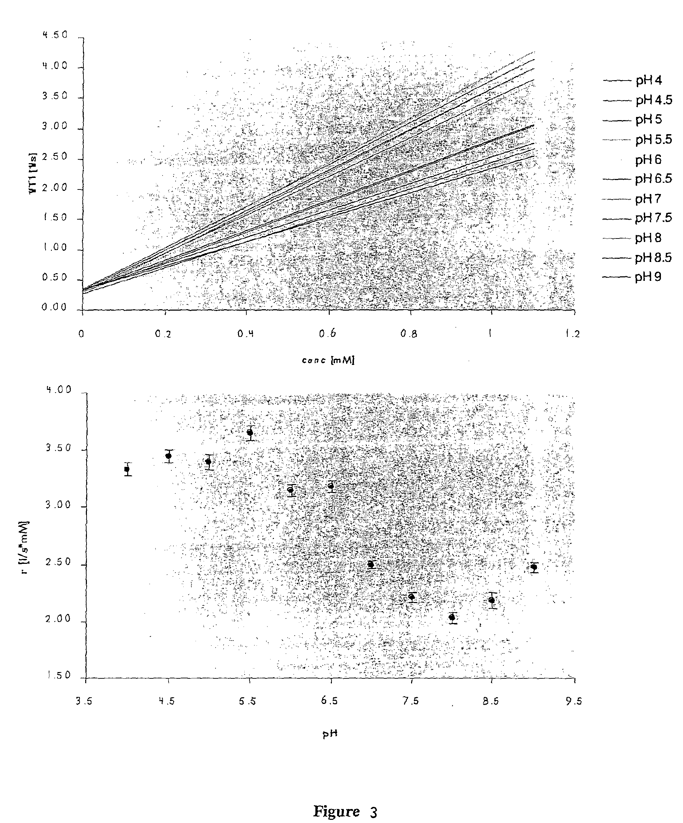

Preliminary data on relaxation studies on the effect of [Ca⁺²] and pH of complexes derived from **4** and **5** are modified in physiological range in the presence and absence of ion/pH is 20% to 143% respectively. The modification of relaxation is significantly higher with pH change.
1. The relaxation rate of gadolinium complexes of **4** and **5** at different concentrations (1.0, 0.7, 0.4, and 0.1mM) was measured in absence of calcium ions. The same concentration was used to measure the relaxation rate in the presence of calcium (concentration range from 10⁻⁹ - 10⁻³M ). The relaxation rate was increased by 20%.
2. The relaxation rate of complex **5** as a function of the pH (4.0, 6.0, and 9.0) was measured at different concentrations (1.0, 0.7, 0.4, and 0.1 mM), Figure 3.
3. Further evaluation as a function of pH (6.2 to 7.4) for the same concentration was performed which relaxation rate was modified from pH 7.4 to 6.2 by 143%.

## Claims

1. A chelating agent of formula (I) 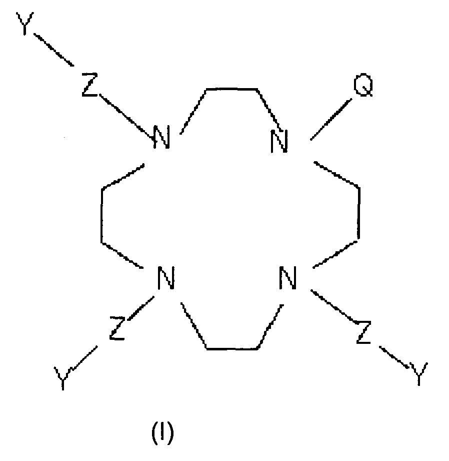 wherein:
Q is a responsive group capable of reversible chelating metal atoms and/or ions, which is not hydrolyzable under physiological conditions, Z is independently a substituted or unsubstituted straight or branched chain alkylene or alkenylene group, and
Y is in each case independently an acidic group,
or a salt thereof.

2. The chelating agent according to claim 1
wherein the chelating capability of Q depends on environmental conditions of pH, enzymes, polyaminoacids and/or cations.

3. The chelating agent according to claim 1 or 2
wherein Q comprises a linking group and an acidic group X.

4. The chelating agent according to any of claims 1 to 3
wherein
Q contains one or more heteroatoms selected from N, O, P and S.

5. The chelating agent according to any of claims 1 to 4
wherein
Q contains a cyclic, e.g. mono or oligocyclic group.

6. The chelating agent according to claim 5
wherein
the cyclic group is a heterocyclic, particularly heteroaryl group.

7. The chelating agent according to any of claims 1 to 6
wherein
Q contains at least one C=C and/or C=N double bond.

8. The chelating agent according to any of claims 1 to 7
wherein
Q contains an electron donating moiety.

9. The chelating agent according to any of claims 1 to 8
wherein
Q is represented by formula (II)
-(CR¹₂)ₙ-CR²=N-A-(CR³₂)ₚ-O-X (II)
wherein n and p are independently integers from 0 to 5,
R¹, R² and R³ independently represent H, OH, amino, carboxyl, sulfo, substituted or unsubstituted alkyl with up to 10 carbon atoms, C₁₋₆ alkoxy, C₆₋₁₄ aryloxy, C₆₋₁₄ arylthio, C₃₋₆ alkenyl, C₃₋₆ alkinyl, C₃₋₆ acyl, C₃₋₆ alkoxycarbonyl, C₆₋₁₄ carbamoyl, C₇₋₁₄ aralkyl, C₆₋₁₄ aryl or C₆₋₁₄ heteroaryl,
X is an acidic group, and
A represents a substituted or unsubstituted ring system with up to 20 carbon atoms and 0-4 hetero atoms selected from the group of N, S, and O.

10. The chelating agent according to claim 9
wherein
Z is C₂H₄, Y is CO₂H, n is 2, p is 1,
R¹, R² and R³ are H and
A is substituted or unsubstituted pyridin.

11. A metal chelate comprising a chelating agent according to any of the previous claims and a metal atom or ion.

12. The metal chelate according to claim 11,
wherein
the metal is selected from the group consisting of transition metals, lanthanides and actinides.

13. The metal chelate according to claim 11 or 12,
wherein
the metal is gadolinium.

14. The metal chelate according to any of claims 11 to 13,
wherein
Q participates in the coordination to the metal.

15. The metal chelate according to claim 14,
wherein
the binding of Q to the metal can be altered by the presence or absence of cations, enzymes and/or polyamido acids and/or by varying the pH.

16. A conjugate comprising a chelating agent according to any one of claims 1 to 10 or a metal chelate according to any of claims 11 to 15 and a biomolecule.

17. A pharmaceutical composition comprising as an active ingredient a chelating agent according to any of claims 1 to 10 or a metal chelate according to any of claims 11 to 15 or a conjugate according to claim 16, optionally together with pharmaceutically acceptable carriers, diluents and/or adjuvants.

18. The composition according to claim 17, for therapeutic or diagnostic applications.

19. The composition according to claim 18, for targeting predetermined sites in an organism.

20. The composition according to any of claims 17 to 19, for use as a contrast agent, particularly in MRI, gamma or positron emission tomography, optical or fluorescence microscopy.

21. Use of a chelating agent according to any of claims 1 to 10 or a metal chelate according to any of claims 11 to 15 or a conjugate according to claim 16 for the manufacture of a contrast agent.

22. The use according to claim 21, wherein the relaxivity of the contrast agent is dependent on the presence and/or amount of cations.

23. The use according to any of claims 21 to 22, wherein the relaxivity of the contrast agent is dependent on the pH.
